# EUROPEAN PATENT APPLICATION

(11) **EP 4 194 021 A2**
(43) Date of publication of application: **14.06.2023**
(21) Application number: 22209652.1
(22) Date of filing: 25.11.2022
(51) Int. Cl.: A61M 1/00, A61M 3/02

(54) **SURGICAL DEVICES, SYSTEMS, AND METHODS FACILITATING MULTIPLE FLOW PATH FLUID MANAGEMENT**

(30) Priority: 13.12.2021 US 202117549758
(71) Applicant: Medtronic Xomed, Inc., Jacksonville, FL 32216 (US)
(72) Inventor: Lahey, Clint N.M., Jacksonville, 32216 (US); Nguyen, Thoai D., Jacksonville, 32216 (US); Slenker, Dale E., Jacksonville, 32216 (US); Morley, Alexander J., Jacksonville, 32216 (US); Bates, Kathleen J., Boulder, 80301 (US); Mowlai-Ashtiani, Ali, Jacksonville, 32216 (US); Malang, Keith W., Boulder, 80301 (US); Hrenchir, Paul, Boulder, 80301 (US)
(74) Representative: Zimmermann & Partner Patentanwälte mbB

(57) **Abstract**

A fluid management system includes a control console and a cassette. The control console includes a housing, a cassette bay defined within the housing, and an actuation mechanism disposed within the housing and including a plurality of actuators. The cassette is insertable into the cassette bay of the control console and includes a chassis supporting a plurality of fluid flow paths through the cassette, and an outer housing defining at least one access opening providing access to the plurality of fluid flow paths. With the cassette positioned within the cassette bay, each actuator of the plurality of actuators is aligned with an access opening of the at least one access opening to enable each actuator, upon actuation thereof, to close a corresponding fluid flow path of the plurality of fluid flow paths.

## Description

### FIELD

The present disclosure relates to surgical fluid management and, more specifically, to surgical devices, systems, and methods facilitating multiple flow path fluid management.

### BACKGROUND

Various surgical devices and systems utilize fluid management to facilitate performing a surgical task such as, for example, to irrigate a treatment site, aspirate a treatment site, clean a surgical device, wash a treatment site, clear a field of view, cool a surgical device, etc. Some nonlimiting examples of these types of surgical devices include micro-deb riders, surgical drills, suction-irrigators, tissue shavers, endoscopes, balloon or other catheters, energy-based devices, and the like.

Some surgical devices require fluid management of multiple fluid flow paths, for example, to enable two or more of irrigation, aspiration, cleaning, lavage, clearing, cooling, etc. As the number of fluid flow paths increases, additional tubing and/or valve structures are required to enable selective and independent management of each fluid flow path. This challenge is complicated by the desire to avoid contamination of capital equipment with fluid, e.g., avoiding fluid contact with the fluid management console and, instead, confining fluid travel to within the disposable, e.g., the cassette connectable to the fluid management console.

### SUMMARY

The terms "about," substantially," and the like, as utilized herein, are meant to account for manufacturing, material, environmental, use, and/or measurement tolerances and variations, and in any event may encompass differences of up to 10%. Further, to the extent consistent, any of the aspects described herein may be used in conjunction with any or all of the other aspects described herein.

Provided in accordance with aspects of the present disclosure is a fluid management system including a control console and a cassette. The control console includes a housing, a cassette bay defined within the housing, and an actuation mechanism disposed within the housing. The actuation mechanism includes a plurality of actuators. The cassette is insertable into the cassette bay of the control console and includes a chassis supporting a plurality of fluid flow paths through the cassette, and an outer housing defining at least one access opening providing access to the plurality of fluid flow paths. With the cassette positioned within the cassette bay, each actuator of the plurality of actuators is aligned with an access opening of the at least one access opening to enable each actuator, upon actuation thereof, to close a corresponding fluid flow path of the plurality of fluid flow paths.

In an aspect of the present disclosure, each actuator of the plurality of actuators is a plunger. In such aspects, each plunger may be biased towards an un-actuated position.

In another aspect of the present disclosure, the actuation mechanism includes a plurality of solenoids. Each solenoid of the plurality of solenoids is configured to selectively actuate one of the actuators of the plurality of actuators.

In still another aspect of the present disclosure, the actuation mechanism includes a cam roller configured to rotate to a plurality of different orientations to actuate the plurality of actuators in different combinations.

In yet another aspect of the present disclosure, each orientation of the plurality of different orientations of the cam roller corresponds to a different combination of closed and open fluid flow paths of the plurality of fluid flow paths. In aspects, the orientations provide every possible combination.

In still yet another aspect of the present disclosure, the plurality of fluid flow paths includes first, second, and third fluid flow paths and the plurality of actuators includes first, second, and third actuators.

In another aspect of the present disclosure, the cam roller includes first, second, and third sections along a length thereof. Each of the first, second, and third sections includes a different pattern of protrusions and no protrusions radially disposed around a circumference thereof such that each orientation of the cam roller provides a different combination of protrusions and no protrusion along the first, second, and third sections.

In yet another aspect of the present disclosure, the first, second, and third sections are positioned adjacent the first, second, and third actuators, respectively. In such aspects, for each orientation of the cam roller, the presence of a protrusion in one of the first, second, or third section actuates the respective first, second, or third actuator, and wherein no protrusion in one of the first, second, or third section does not actuate the respective first, second, or third actuator.

In another aspect of the present disclosure, the control console further includes a rotary motor configured to drive rotation of the cam roller.

A fluid management cassette insertable into a cassette bay of a control console and provided in accordance with aspects of the present disclosure includes a chassis supporting a first fluid flow path, a second fluid flow path, and a third fluid flow path; a face plate including a plurality of input fluid connection ports each providing input to one of the first, second, or third fluid flow paths, and a plurality of output fluid connection ports each receiving output from one of the first, second, or third fluid flow paths; and an outer housing cooperating with the face plate to define an enclosure. The outer housing defines at least one access opening providing access to the first, second, and third fluid flow paths to enable selective closing of fluid flow through any combination of the first, second, or third fluid flow paths.

In an aspect of the present disclosure, the fluid management cassette further includes a pump mounted on the chassis and operably disposed along at least the first fluid flow path and the second fluid flow path.

In another aspect of the present disclosure, the first and second fluid flow paths share a common input fluid connection port of the plurality of input fluid connection ports, common tubing to the pump, and separate tubing from the pump to different output fluid connection ports of the plurality of output fluid connection ports.

In still another aspect of the present disclosure, the third fluid flow path includes tubing connecting another input fluid connection port of the plurality of input fluid connection ports and another output fluid connection port of the plurality of output fluid connection ports.

In yet another aspect of the present disclosure, the outer housing is further configured to receive a drive rotor and/or defines the at least one access opening through a side or a top thereof.

Another fluid management system provided in accordance with aspects of the present disclosure includes first, second, and third fluid flow paths each capable of having an open state or a closed state thereby defining eight different combinations of states of the first, second, and third fluid flow paths. The system further includes first, second, and third actuators operably positioned relative to the first, second, and third fluid flow paths, respectively, and selectively actuatable to close the respective first, second, and third fluid flow paths. In addition, the system includes a cam roller positioned adjacent the first, second, and third actuators and having eight different orientations. In each different orientation of the eight different orientations, the cam roller actuates a different combination of the first, second, and third actuators such that each different orientation of the eight different orientations achieves one of the eight different combinations of states of the first, second, and third fluid flow paths.

In an aspect of the present disclosure, the cam roller is mounted on a drive rotor and a motor is configured to drive rotation of the drive rotor to thereby achieve a desired orientation of the cam roller.

In another aspect of the present disclosure, the first, second, and third fluid flow paths are defined through a cassette releasably engagable with a control console housing the first, second, and third actuators and the cam roller.

In still another aspect of the present disclosure, the cassette further includes a pump operably disposed along at least one of the first, second, or third fluid flow paths.

### BRIEF DESCRIPTION OF THE DRAWINGS

Various aspects and features of the present disclosure are described hereinbelow with reference to the drawings wherein:
FIG. 1 is a perspective view of a surgical system provided in accordance with aspects of the present disclosure;
FIG. 2A is a perspective view of a cassette of the surgical system of FIG. 1 configured for use with the control console of the surgical system of FIG. 1;
FIGS. 2B and 2C are first and second side, perspective views, respectively, of the cassette of FIG. 2A with the outer housing thereof removed to illustrate internal components and features thereof;
FIG. 3A is a perspective view of the cassette of FIG. 2A operably positioned within a cassette bay and relative to the actuation mechanism of the control console of FIG. 1 for selectively managing the various fluid flow paths of the cassette in accordance with aspects of the present disclosure;
FIG. 3B is a perspective, transverse cross-sectional view of the cassette of FIG. 2A operably positioned within the cassette bay and relative to the actuation mechanism of FIG. 3A in accordance with aspects of the present disclosure;
FIG. 4A is a perspective view of another cassette bay and actuation mechanism configured for use with the control the control console of FIG. 1 in accordance with aspects of the present disclosure;
FIG. 4B is a perspective view of a cassette configured for operable positioned within and relative to the cassette bay and actuation mechanism of FIG. 4A in accordance with aspects of the present disclosure;
FIG. 5A and 5B are first and second transverse cross-sectional views of the cassette of FIG. 4B operably positioned within the cassette bay and relative to the actuation mechanism of FIG. 4A in accordance with aspects of the present disclosure;
FIG. 6 is a perspective views of the cam roller of the actuation mechanism of FIG. 4A in accordance with aspects of the present disclosure; and
FIG. 7 is a table illustrating various rotational orientations of the cam roller of FIG. 6 and corresponding fluid flow path states for each rotational orientation in accordance with aspects of the present disclosure.

### DETAILED DESCRIPTION

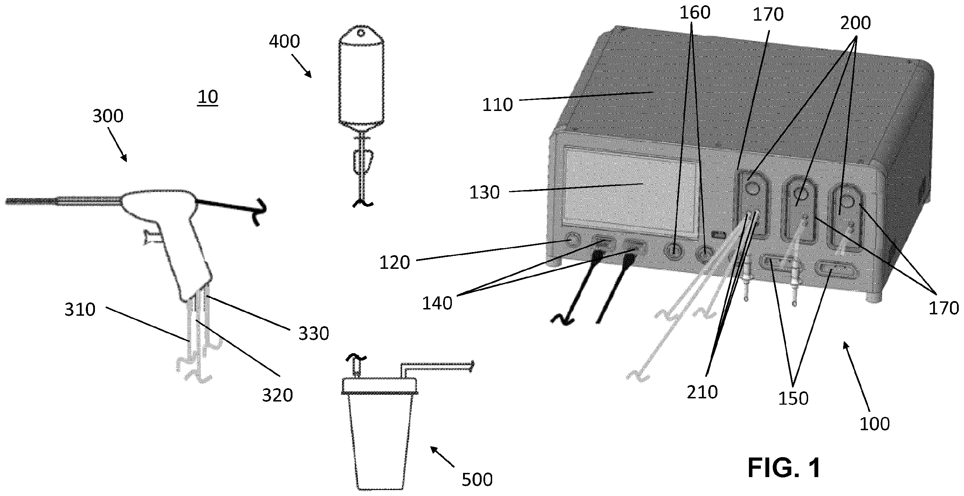

Referring to FIG. 1, a surgical system 10 provided in accordance with the present disclosure generally includes: a control console 100; one or more fluid management cassettes 200 configured for releasable engagement with control console 100; one or more surgical instruments 300 configured to be powered, controlled, energized, supplied fluid, and/or supplied vacuum by control console 100; one or more fluid sources 400; and/or one or more fluid collection canisters 500. Although plural fluid management cassettes 200, surgical instruments 300, fluid sources 400, and fluid collection canisters 500 are contemplated, surgical system 10 is described below with reference to only one fluid management cassette 200, one surgical instrument 300, one fluid source 400, and one fluid collection canister 500 for the purposes of brevity and understanding. Likewise, although control console 100 may include plural similar components, each of such may be described in the singular hereinbelow for the purposes of brevity and understanding.

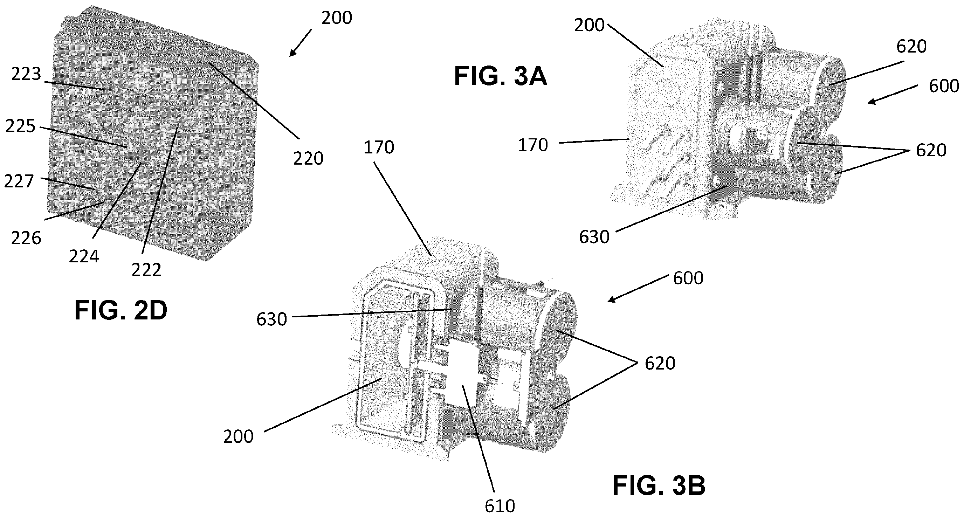

Control console 100 includes: a housing 110, a power button 120; a graphical user interface 130, one or more power ports 140 for powering and controlling connected powered surgical instrument(s), one or more energy ports 150 for providing surgical energy, e.g., nonpolar, bipolar, microwave, ultrasonic, thermal, light, and/or other surgical energy, to connected energy-based surgical instrument(s), one or more additional ports 160; and a plurality of cassette bays 170. Control console 100 further includes one or more central processing units (CPU's) and/or microcontroller units (MCU's), power generating and control hardware, surgical energy generating and control hardware, and any other suitable hardware and corresponding firmware/software stored thereon for operating and controlling operation of surgical instruments 300 connected thereto. Control console 100 further includes an actuation mechanism 600, 1600 (FIGS. 3A-3B and FIGS. 4A and 5A-5B, respectively) operably positioned relative to a corresponding cassette bay 170 to control the various fluid paths defined through fluid management cassette 200 when fluid management cassette 200 is received within cassette bay 170. One of the CPU's and/or MCU's may also control actuation mechanism 600, 1600 according to a particular control program selected (e.g., via graphical user interface 130), according to user-actuation of one or more controls associated with surgical instrument 300, in response to sensed feedback, and/or in any other suitable manner.

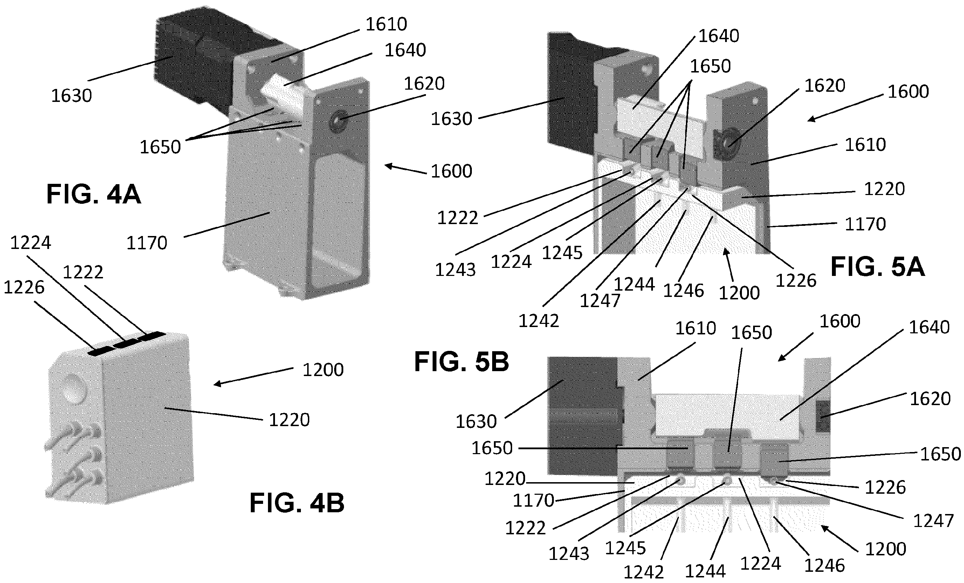

Fluid management cassette 200, as noted above, is receivable within cassette bay 170 to operably couple the fluid flow paths defined therethrough with a corresponding actuation mechanism 600, 1600 (FIGS. 3A-3B and FIGS. 4A and 5A-5B, respectively) to enable independent control of the various fluid flow paths, e.g., to close, open, and/or control fluid flow along the various fluid flow paths. Fluid management cassettes 200, 1200 (FIGS. 4B-5B) suitable for use as fluid management cassette 200 in accordance with the present disclosure are described in greater detail below, as are corresponding actuation mechanisms 600, 1600 (FIGS. 3A-3B and FIGS. 4A and 5A-5B, respectively). Regardless of the particular configuration, fluid management cassettes 200, 1200 (FIGS. 4B-5B) may include a plurality of external tubing ports 210 to enable connection of suitable tubing to fluidly couple fluid management cassettes 200, 1200 (FIGS. 4B-5B) with one or more fluid flow paths associated with surgical instrument 300, fluid source 400, and/or fluid collection canister 500.

Continuing with reference to FIG. 1, surgical instrument 300, as noted above, may be powered, controlled, energized, supplied fluid, and/or supplied vacuum by control console 100. Surgical instrument 300 may be configured as, for example and without limitation, one or more of a micro-debrider, surgical drill, suction-irrigator, tissue shaver, endoscope, sheath for an endoscope (e.g., a lens-cleaning sheath), balloon or other catheter, energy-based device, fluid-cooled device, etc. In some aspects, surgical instrument 300 defines at least three fluid flow paths 310, 320, 330 therethrough or otherwise associated therewith such as, for example, an irrigation fluid flow path 310, a lavage fluid flow path 320, and a suction fluid flow path 330.

Fluid source 400, e.g., an IV-style fluid bag, is fluidly coupled to one or more fluid flow paths defined within fluid management cassette 200. Fluid management cassette 200, as noted above, is connected to surgical instrument 300 and is configured to enable control console 100 to control the delivery of fluid from fluid source 400 to the surgical instrument 300. More specifically, as an example, fluid management cassette 200 may operably couple fluid source 400 with irrigation fluid flow path 310 and lavage fluid flow path 320 of surgical instrument 300 to enable control console 100 to selectively supply fluid along fluid flow paths 310, 320.

Fluid collection canister 500 is fluidly coupled between the surgical instrument 300 and fluid management cassette 200 to enable control console 100 to control the suctioning of fluid from the surgical instrument 300 into the fluid collection canister 500, e.g., along suction fluid flow path 330.

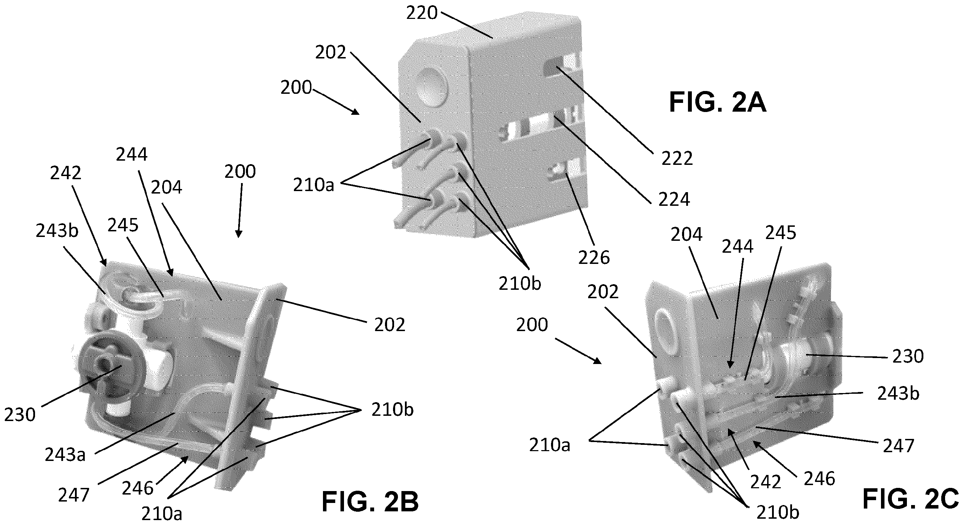

Turning to FIGS. 2A-2C, fluid management cassette 200 in accordance with aspects of the present disclosure is shown. Fluid management cassette 200 includes a face plate 202 supported on a chassis 204 and defining the plurality of external tubing ports 210 (FIG. 1), e.g., a plurality of input tubing ports 210a and a plurality of output tubing ports 210b, that enable connection of fluid management cassette 200 with fluid source 400 (FIG. 1), surgical instrument 300 (FIG. 1), and/or fluid collection canister 500 (FIG. 1). Fluid management cassette 200 further includes an outer housing 220 that cooperates with face plate 202 to enclose the internal components and features of fluid management cassette 200. Outer housing 220 defines a plurality of side openings 222, 224, 226 to enable control console 100 (FIG. 1) to selectively open, close, and/or otherwise control the fluid flow paths defined through fluid management cassette 200, as detailed below. Outer housing 220 also defines one or more rear receptacles (not explicitly shown) configured to enable operable coupling of a pump drive rotor (not explicitly shown) of control console 100 (FIG. 1) with pump 230 of fluid management cassette 200 to drive pump 230 of fluid management cassette 200.

In aspects, fluid management cassette 200 defines three fluid flow paths 242, 244, 246 with two of the fluid flow paths 242, 244 configured as fluid inflow paths and the third fluid flow path 246 configured as a fluid outflow path. However, greater than three fluid flow paths and/or various different combinations of inflow and/or outflow paths are also contemplated. First fluid inflow path 242 includes tubing 243a connecting one of the input tubing ports 210a to pump 230 and tubing 243b connecting a first output of pump 230 with one of the output tubing ports 210b. Second fluid inflow path 244 shares tubing 243a with first inflow path 244 and includes tubing 245 that connects a second output of pump 230 with another one of the output tubing ports 210b. Fluid source 400 (FIG. 1) can be connected to the input tubing port 210a to thus supply fluid for selective pumping along flow paths 242, 244 and out the corresponding output tubing port 210b. However, in other aspects, flow paths 242, 244 need not share tubing or a single fluid source 400 (FIG. 1). Surgical instrument 300 (FIG. 1) and, more specifically, fluid flow paths 310 and 320 thereof can be connected to the output tubing ports 210b to enable the supply of fluid thereto for irrigation and lavage, respectively.

Fluid outflow path 246 includes tubing 247 connecting another one of the input tubing ports 210a to another one of the output tubing ports 210b such that a vacuum source (not explicitly shown) can be connected to the input tubing port 210a and fluid path 330 of surgical instrument 300 (FIG. 1) can be connected to the output tubing ports 210b (by way of fluid collection canister 500 (FIG. 1)) to provide surgical instrument 300 (FIG. 1) with suction capabilities. Other suitable fluid connections, flow paths, and pump configurations for fluid supply and/or return are also contemplated.

Continuing with reference to FIGS. 2A-2C, side openings 222, 224, 226 of outer housing 220 provide access to tubing 243b, 245, 247 to enable actuation mechanism 600 (FIGS. 3A-3B) to selectively leave open, fully close, or partially close the internal lumens defined through tubing 243b, 245, 247, thereby selectively controlling irrigation, lavage, and/or suction through surgical instrument 300 (FIG. 1).

With reference to FIGS. 3A and 3B, in conjunction with FIGS. 2A-2C, actuation mechanism 600 of control console 100 (FIG. 1) includes a plurality of actuators 610, e.g., three actuators 610, and a plurality of drivers 620, e.g., three drivers 620, to selectively and independently drive the actuators 610. Actuation mechanism 600 further includes a support 630 mounted on cassette bay 170 and operably retaining actuators 610 and drivers 620 in position relative thereto. In aspects, actuators 610 may be plungers selectively deployable into and retractable from cassette bay 170. In such aspects, drivers 620 may be solenoids configured to selectively deploy and retract (or allow for spring-biased retraction of) actuators 610.

Upon receipt of fluid management cassette 200 within cassette bay 170, each side opening 222, 224, 226 of outer housing 220 of fluid management cassette 200 is aligned with one of the actuators 610 of actuation mechanism 600. In this manner, each actuator 610 of actuation mechanism 600 is aligned with tubing 243b, 245, 247 of one of fluid flow paths 242, 244, 246 of fluid management cassette 200, respectively. Thus, drivers 620 can be controlled to selectively deploy or retract the corresponding actuators 610 to thereby selectively leave open, fully close, or partially close the internal lumens defined through tubing 243b, 245, 247 and, thus, selectively control irrigation, lavage, and/or suction through surgical instrument 300 (FIG. 1). With respect to fluid inflow paths 242, 244, this configuration enables independent control of fluid inflow paths 242, 244 using a single pump 230 (driven by the pump rotor (not shown) of control console 100 (FIG. 1)). For example, with pump 230 activated, either, both, or neither of irrigation and lavage can be provided. Likewise, suction can be controlled at control console 100 (FIG. 1) via the appropriate actuator 610 selectively leaving open, fully closing, or partially closing the internal lumen defined through tubing 247.

Referring to FIG. 2D, in conjunction with FIGS. 3A and 3B, in aspects, rather than fully open side openings 222, 224, 226, outer housing 220 of fluid management cassette 200 may include actuation levers 223, 225, 227, respectively, e.g., in the form of lever arms cut-out from outer housing 220 and operably positioned within side openings 222, 224, 226 via living hinges. Actuation levers 223, 225, 227 thus function as intermediary structures such that, when an actuator 610 is actuated and extends into contact with the corresponding actuation lever 223, 225, 227, the corresponding actuation lever 223, 225, 227 is depressed into outer housing 220 to close, or partially close the internal lumens defined through tubing 243b, 245, 247, respectively (see FIGS. 2A and 2B). Thus, actuators 610 may directly or indirectly interact with tubing 243b, 245, 247 (FIGS. 2A and 2B) to selectively leave open, close, or partially close fluid flow paths 242, 244, 246 (FIGS. 2B and 2C).

Turning to FIGS. 4A-7, another fluid management cassette 1200 and actuation mechanism 1600 provided in accordance with the present disclosure and configured for use with surgical system 10 (FIG. 1) or any other suitable surgical system are described. Fluid management cassette 1200 is similar to and may include any of the features of fluid management cassette 200 (FIGS. 2A-2C) except as explicitly contradicted below.

Fluid management cassette 1200 includes top openings 1222, 1224, 1226 defined within outer housing 1220 thereof. Further, the tubing 1243, 1245, 1247 associated with the first, second, and third fluid flow paths 1242, 1244, 1246 through fluid management cassette 1200, respectively, are routed to be exposed within top openings 1222, 1224, 1226, respectively. Other positions of top openings 1222, 1224, 1226 (e.g., on the sides, back, and/or other locations of cassette 1200) and/or routing configurations of tubing 1243, 1245, 1247 are also contemplated.

Actuation mechanism 1600 includes a frame 1610 formed with or attached to cassette bay 1170. Frame 1610 rotatably supports a rotor 1620 connected to a rotary motor 1630, e.g., a stepper motor or other suitable motor, configured to selectively drive rotation of rotor 1620 to a desired rotational orientation. A cam roller 1640 is disposed about rotor 1620 within frame 1610 such that rotation of rotor 1620 similarly rotates cam roller 1640. Frame 1610 further includes a plurality, e.g., three, spring-loaded actuators 1650, e.g., plungers, supported therein adjacent cassette bay 1170. Each spring-loaded actuator 1650 is biased towards a retracted or un-actuated position wherein the actuator 1650 does not extend into cassette bay 1170 and is movable from the retracted position to an extended position wherein the actuator 1650 protrudes at least partially into cassette bay 1170. Cam roller 1640 is configured and positioned to selectively urge none, one, some, or all of actuators 1650 from the retracted position(s) thereof to the extended position(s) thereof depending upon the orientation of cam roller 1640, as detailed below.

Upon receipt of fluid management cassette 1200 within cassette bay 1170, each top opening 1222, 1224, 1226 of outer housing 1220 of fluid management cassette 1200 is aligned with one of the actuators 1650 of actuation mechanism 1600. In this manner, each actuator 1650 of actuation mechanism 1600 is aligned with tubing 1243, 1245, 1247 of one of fluid flow paths 1242, 1244, 1246 of fluid management cassette 1200. Thus, cam roller 1640 can be driven to rotate to a desired rotational orientation to selectively deploy or allow spring-biased retraction of the corresponding actuator 1650 to thereby selectively leave open or close the internal lumens defined through tubing 1243, 1245, 1247 and, thus, to selectively control irrigation, lavage, and/or suction through surgical instrument 300 (FIG. 1).

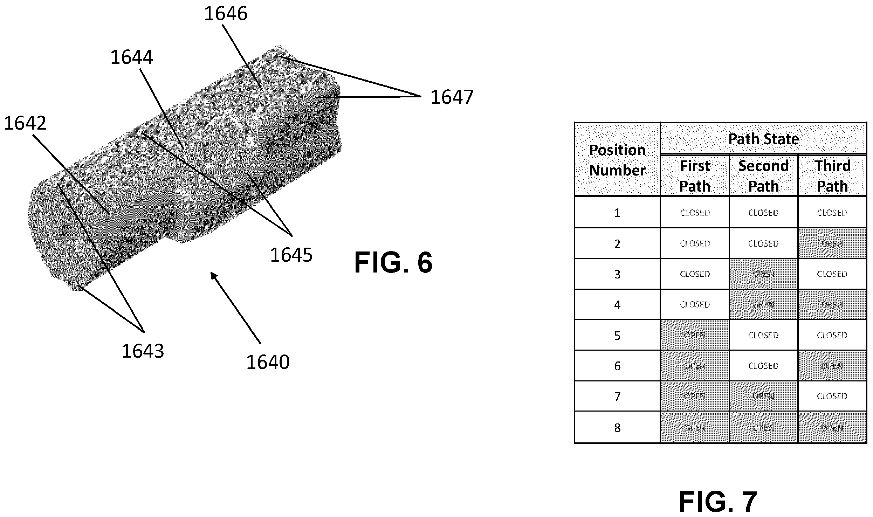

With particular reference to FIG. 6, cam roller 1640 includes first, second, and third longitudinal sections 1642, 1644, 1646 defined sequentially along the length thereof. Each longitudinal section 1642, 1644, 1646 is configured for deploying or allowing retraction of one of the actuators 1650 (FIGS. 4, 5A, and 5B). More specifically, each section 1642, 1644, 1646 includes a plurality of protrusions 1643, 1645, 1647 spaced radially about the circumference thereof according to different patterns. As a result, for any given rotational orientation of cam roller 1640, a specific longitudinal pattern is achieved depending upon whether or not each longitudinal section 1642, 1644, 1646 includes a protrusion 1643, 1645, 1647 in that particular orientation.

Referring also to FIG. 7, for example, cam roller 1640 may define eight (8) different rotational orientations, each designated as "position number" in the table illustrated in FIG. 7, wherein each orientation constitutes approximately 45 degrees of outer circumference of cam roller 1640. For each rotational orientation: the presence or absence of a protrusion 1643 in first section 1642 determines whether the corresponding actuator 1650 (FIGS. 5A and 5B) is deployed or remains retracted and, thus, determines whether irrigation is enabled or prevented; the presence or absence of a protrusion 1645 in second section 1644 determines whether the corresponding actuator 1650 (FIGS. 5A and 5B) is deployed or remains retracted and, thus, determines whether lavage is enabled or prevented; and the presence or absence of a protrusion 1647 in third section 1646 determines whether the corresponding actuator 1650 (FIGS. 5A and 5B) is deployed or remains retracted and, thus, determines whether suction is enabled or prevented. Providing eight (8) positions each with a different pattern of protrusions 1643, 1645, 1647 or the lack thereof enables all possible combinations of activation and deactivation of irrigation, lavage, and suction, as indicated in the table of FIG. 7.

Other suitable configurations and/or number of rotational orientations are also contemplated. For example, half-protrusions may be provided on cam roller 1640 and additional rotational orientations may be provided to enable all possible combinations of activation and deactivation of irrigation, lavage, and suction and partial activation of at least one of irrigation, lavage, and suction.

It will be understood that various modifications may be made to the aspects and features disclosed herein. Therefore, the above description should not be construed as limiting, but merely as exemplifications of various aspects and features. Those skilled in the art will envision other modifications within the scope and spirit of the claims appended thereto.

A fluid management system includes a control console and a cassette. The control console includes a housing, a cassette bay defined within the housing, and an actuation mechanism disposed within the housing and including a plurality of actuators. The cassette is insertable into the cassette bay of the control console and includes a chassis supporting a plurality of fluid flow paths through the cassette, and an outer housing defining at least one access opening providing access to the plurality of fluid flow paths. With the cassette positioned within the cassette bay, each actuator of the plurality of actuators is aligned with an access opening of the at least one access opening to enable each actuator, upon actuation thereof, to close a corresponding fluid flow path of the plurality of fluid flow paths.

Further disclosed herein is the subject-matter of the following clauses:
1. A fluid management system, comprising:
   a control console, including:
      a housing;
      a cassette bay defined within the housing; and
      an actuation mechanism disposed within the housing, the actuation mechanism including a plurality of actuators; and
   a cassette insertable into the cassette bay of the control console, the cassette including:
      a chassis supporting a plurality of fluid flow paths through the cassette; and
      an outer housing defining a plurality of access openings providing access to the plurality of fluid flow paths,
   wherein, with the cassette positioned within the cassette bay, each actuator of the plurality of actuators is aligned with an access opening of the plurality of access openings to enable each actuator, upon actuation thereof, to close a corresponding fluid flow path of the plurality of fluid flow paths.
2. The fluid management system according to clause 1, wherein each actuator of the plurality of actuators is a plunger.
3. The fluid management system according to clause 1 or 2, wherein each plunger is biased towards an un-actuated position.
4. The fluid management system according to clause 1 or according to any of the preceding clauses, wherein the actuation mechanism includes a plurality of solenoids, each solenoid of the plurality of solenoids configured to selectively actuate one of the actuators of the plurality of actuators.
5. The fluid management system according to clause 1 or according to any of the preceding clauses, wherein the actuation mechanism includes a cam roller configured to rotate to a plurality of different orientations to actuate the plurality of actuators in different combinations.
6. The fluid management system according to clause 5 or according to any of the preceding clauses, wherein each orientation of the plurality of different orientations of the cam roller corresponds to a different combination of closed and open fluid flow paths of the plurality of fluid flow paths.
7. The fluid management system according to clause 5 or according to any of the preceding clauses, wherein the plurality of fluid flow paths includes first, second, and third fluid flow paths, and wherein the plurality of actuators includes first, second, and third actuators.
8. The fluid management system according to clause 7 or according to any of the preceding clauses, wherein the cam roller includes first, second, and third sections along a length thereof, each of the first, second, and third sections including a different pattern of protrusions and no protrusions radially disposed around a circumference thereof such that each orientation of the cam roller provides a different combination of protrusions and no protrusion along the first, second, and third sections.
9. The fluid management system according to clause 8 or according to any of the preceding clauses, wherein the first, second, and third sections are positioned adjacent the first, second, and third actuators and wherein, for each orientation of the plurality of different orientations of the cam roller, the presence of a protrusion in one of the first, second, or third section actuates the respective first, second, or third actuator, and no protrusion in the first, second, or third section does not actuate the respective first, second, or third actuator.
10. The fluid management system according to clause 5 or according to any of the preceding clauses, wherein the control console further includes a rotary motor configured to drive rotation of the cam roller.
11. A fluid management cassette insertable into a cassette bay of a control console, the cassette comprising:
   a chassis supporting a first fluid flow path, a second fluid flow path, and a third fluid flow path;
   a face plate including a plurality of input fluid connection ports each providing input to one of the first, second, or third fluid flow paths, and a plurality of output fluid connection ports each receiving output from one of the first, second, or third fluid flow paths; and
   an outer housing cooperating with the face plate to define an enclosure, the outer housing defining at least one access opening providing access to the first, second, and third fluid flow paths to enable selective closing of fluid flow through any combination of the first, second, or third fluid flow paths.
12. The fluid management cassette according to clause 11, further comprising a pump mounted on the chassis, the pump operably disposed along at least the first fluid flow path and the second fluid flow path.
13. The fluid management cassette according to clause 11 or 12, wherein the first and second fluid flow paths share a common input fluid connection port of the plurality of input fluid connection ports, common tubing to the pump, and separate tubing from the pump to different output fluid connection ports of the plurality of output fluid connection ports.
14. The fluid management cassette according to clause 11 or according to any of the clauses 11 to 13, wherein the third fluid flow path includes tubing connecting another input fluid connection port of the plurality of input fluid connection ports and another output fluid connection port of the plurality of output fluid connection ports.
15. The fluid management cassette according to clause 11 or according to any of the clauses 11 to 14, wherein the outer housing is further configured to receive a drive rotor.
16. The fluid management cassette according to clause 11 or according to any of the clauses 11 to 15, wherein the at least one access opening is defined through a side of the outer housing or a top of the outer housing.
17. A fluid management system, comprising:
   first, second, and third fluid flow paths each capable of having an open state or a closed state thereby defining eight different combinations of states of the first, second, and third fluid flow paths;
   first, second, and third actuators operably positioned relative to the first, second, and third fluid flow paths, respectively, and selectively actuatable to close the respective first, second, and third fluid flow paths; and
   a cam roller positioned adjacent the first, second, and third actuators and having eight different orientations, wherein in each different orientation of the eight different orientations, the cam roller actuates a different combination of the first, second, and third actuators such that each different orientation of the eight different orientations achieves one of the eight different combinations of states of the first, second, and third fluid flow paths.
18. The fluid management system according to clause 17, wherein the cam roller is mounted on a drive rotor, and wherein a motor is configured to drive rotation of the drive rotor to thereby achieve a desired orientation of the cam roller.
19. The fluid management system according to clause 17 or 18, wherein the first, second, and third fluid flow paths are defined through a cassette releasably engagable with a control console housing the first, second, and third actuators and the cam roller.
20. The fluid management system according to clause 19 or according to any of the clauses 17 to 19, wherein the cassette further includes a pump operably disposed along at least one of the first, second, or third fluid flow paths.

## Claims

1. A fluid management system, comprising:
a control console, including:
a housing;
a cassette bay defined within the housing; and
an actuation mechanism disposed within the housing, the actuation mechanism including a plurality of actuators; and
a cassette insertable into the cassette bay of the control console, the cassette including:
a chassis supporting a plurality of fluid flow paths through the cassette; and
an outer housing defining a plurality of access openings providing access to the plurality of fluid flow paths,
wherein, with the cassette positioned within the cassette bay, each actuator of the plurality of actuators is aligned with an access opening of the plurality of access openings to enable each actuator, upon actuation thereof, to close a corresponding fluid flow path of the plurality of fluid flow paths.

2. The fluid management system according to claim 1, wherein each actuator of the plurality of actuators is a plunger, particularly wherein each plunger is biased towards an un-actuated position.

3. The fluid management system according to claim 1 or 2, wherein the actuation mechanism includes a plurality of solenoids, each solenoid of the plurality of solenoids configured to selectively actuate one of the actuators of the plurality of actuators.

4. The fluid management system according to one of the claims 1 to 3, wherein the actuation mechanism includes a cam roller configured to rotate to a plurality of different orientations to actuate the plurality of actuators in different combinations.

5. The fluid management system according to one of the claims 1 to 4, wherein each orientation of the plurality of different orientations of the cam roller corresponds to a different combination of closed and open fluid flow paths of the plurality of fluid flow paths.

6. The fluid management system according to one of the claims 1 to 5, wherein the plurality of fluid flow paths includes first, second, and third fluid flow paths, and wherein the plurality of actuators includes first, second, and third actuators.

7. The fluid management system according to one of the claims 1 to 6, wherein the cam roller includes first, second, and third sections along a length thereof, each of the first, second, and third sections including a different pattern of protrusions and no protrusions radially disposed around a circumference thereof such that each orientation of the cam roller provides a different combination of protrusions and no protrusion along the first, second, and third sections.

8. The fluid management system according to one of the claims 1 to 7, wherein the first, second, and third sections are positioned adjacent the first, second, and third actuators and wherein, for each orientation of the plurality of different orientations of the cam roller, the presence of a protrusion in one of the first, second, or third section actuates the respective first, second, or third actuator, and no protrusion in the first, second, or third section does not actuate the respective first, second, or third actuator.

9. The fluid management system according to one of the claims 1 to 8, wherein the control console further includes a rotary motor configured to drive rotation of the cam roller.

10. A fluid management cassette insertable into a cassette bay of a control console, the cassette comprising:
a chassis supporting a first fluid flow path, a second fluid flow path, and a third fluid flow path;
a face plate including a plurality of input fluid connection ports each providing input to one of the first, second, or third fluid flow paths, and a plurality of output fluid connection ports each receiving output from one of the first, second, or third fluid flow paths; and
an outer housing cooperating with the face plate to define an enclosure, the outer housing defining at least one access opening providing access to the first, second, and third fluid flow paths to enable selective closing of fluid flow through any combination of the first, second, or third fluid flow paths.

11. The fluid management cassette according to claim 10, further comprising a pump mounted on the chassis, the pump operably disposed along at least the first fluid flow path and the second fluid flow path, particularly wherein the first and second fluid flow paths share a common input fluid connection port of the plurality of input fluid connection ports, common tubing to the pump, and separate tubing from the pump to different output fluid connection ports of the plurality of output fluid connection ports.

12. The fluid management cassette according to one of the claims 10 to 11, wherein the third fluid flow path includes tubing connecting another input fluid connection port of the plurality of input fluid connection ports and another output fluid connection port of the plurality of output fluid connection ports.

13. The fluid management cassette according to one of the claims 10 to 12, wherein the outer housing is further configured to receive a drive rotor,
and/or
wherein the at least one access opening is defined through a side of the outer housing or a top of the outer housing.

14. A fluid management system, comprising:
first, second, and third fluid flow paths each capable of having an open state or a closed state thereby defining eight different combinations of states of the first, second, and third fluid flow paths;
first, second, and third actuators operably positioned relative to the first, second, and third fluid flow paths, respectively, and selectively actuatable to close the respective first, second, and third fluid flow paths; and
a cam roller positioned adjacent the first, second, and third actuators and having eight different orientations, wherein in each different orientation of the eight different orientations, the cam roller actuates a different combination of the first, second, and third actuators such that each different orientation of the eight different orientations achieves one of the eight different combinations of states of the first, second, and third fluid flow paths.

15. The fluid management system according to claim 14, wherein the cam roller is mounted on a drive rotor, and wherein a motor is configured to drive rotation of the drive rotor to thereby achieve a desired orientation of the cam roller,
and/or
wherein the first, second, and third fluid flow paths are defined through a cassette releasably engagable with a control console housing the first, second, and third actuators and the cam roller, particularly wherein the cassette further includes a pump operably disposed along at least one of the first, second, or third fluid flow paths.
